# EUROPEAN PATENT APPLICATION

(11) **EP 4 483 878 A1**
(43) Date of publication of application: **01.01.2025**
(21) Application number: 23792089.7
(22) Date of filing: 12.04.2023
(51) Int. Cl.: A61K 31/4704, A61P 9/00, A23L 33/10

(54) **COMPOSITION FOR PREVENTING OR TREATING VALVULAR CALCIFICATION**

(30) Priority: 20.04.2022 KR 20220048568
(71) Applicant: Mokpo National University Industry-Academia Cooperation Group, Muan-gun, Jeollanam-do 58554 (KR)
(72) Inventor: OAK, Min Ho, Muan-gun, Jeollanam-do 58568 (KR); KO, Ju Young, Muan-gun, Jeollanam-do 58580 (KR); SHIWAKOTI, Saugat, Muan-gun, Jeollanam-do 58553 (KR)
(74) Representative: Gassner, Birgitta
(86) International application number: PCT/KR2023/004891
(87) International publication number: WO 2023/204511

(57) **Abstract**

The present invention provides a pharmaceutical composition or food composition for preventing, alleviating or treating valvular calcification.

## Description

### BACKGROUND

### Technical Field

The present invention relates to a composition for preventing or treating valvular calcification.

### Background Art

Aortic valve stenosis is a disease in which the aortic valve becomes thick due to inflammation and calcification, causing adhesion and preventing the aortic valve from opening properly. It is one of the three major cardiovascular diseases, along with coronary artery disease and hypertension. If stenosis persists, symptoms of heart failure such as decreased cardiac output and myocardial ischemia may appear, and if the patient does not receive treatment after feeling symptoms, the average mortality rate after 2 years reaches 50%. It is emerging as a common disease and a major cause of death in an aging society, with an incidence of 2% in the population aged 65-74 and more than 6% of the population aged 75 or older showing severe aortic valve stenosis requiring treatment.

Risk factors for aortic valve stenosis include obesity, diabetes, chronic kidney disease, high blood pressure, age, and smoking. It is particularly highly correlated with age, occurring at a higher rate in elderly patients. Although the exact cause of the disease has not been identified yet, it is gradually becoming clear that calcification of valve cells is an important cause. Although several studies have been conducted recently, there is still no target protein that can effectively prevent valvular calcification or substance targeting it, and drug treatment is currently not possible. Aortic valve replacement, which replaces the aortic valve with an artificial valve, is the only treatment, but surgical or percutaneous replacement procedures are not easy because many patients are elderly, and it has disadvantages such as high cost (about 30 million won), increased risk of stroke, possibility of leakage around the valve, and durability problems due to long-term use. Therefore, there is an urgent need to develop a drug treatment that can replace aortic valve replacement, Additionally, because early diagnosis is difficult, development of biomarkers for easy diagnosis is necessary. Consequently, there is an urgent need to discover target proteins that can diagnose and treat aortic valve stenosis.

The surface of the aortic valve leaflets is lined with valvular endothelial cells (VEC), and valvular interstitial cells (VIC) are spread inside. Because heart valves are under strong stress from blood flow, they produce various biochemical signals, matrix proteins, and matrix remodeling enzymes to maintain valve cell tissue homeostasis. It is known that when chronic stress such as aging or calcification occurs, excessive collagen infiltration and matrix protein destruction occur, which increases calcification and causes the valvular interstitial cells to lose their ability to maintain homeostasis. Recent studies have provided various information from a molecular biological perspective on the calcification reaction induced by valve cells, and one of the areas that has received the most attention is the bone formation mechanism by valvular interstitial cells. The calcification process of valvular interstitial cells proposed to date is divided into an initiation phase and a propagation phase. The onset of valve stenosis is thought to be due to damage to the valve endothelium caused by mechanical stress or other risk factors. It has been suggested that low-density lipoprotein (LDL) and lipoprotein(a) [Lp(a)] accumulate and are oxidized after penetrating the subendothelial layer, which may induce a chronic inflammatory response by macrophages, T cells, and mast cells, leading to activation of valvular interstitial cells (VIC). In the propagation phase, activated VICs produce disorganized collagen, leading to fibrosis and progressive valve thickening. Osteoblast-like cells produce calcified vesicles and induce the valvular calcification. It has been proposed that as the valve calcifies, mechanical stress, injury, apoptosis, and osteoblast activation on the valve increase, leading to further calcification, resulting in a vicious cycle.

Research began in earnest in the 2010s and is being conducted primarily overseas, but there is still no clear target protein that can effectively prevent the valvular calcification, so there are no therapeutic drugs. Most of the previous studies have focused on the indirect involvement of interstitial cell calcification in targets and directions similar to atherosclerosis, such as hyperlipidemia, inflammation, and the angiotensin system, rather than on direct changes in proteins, etc., of interstitial cells during the calcification process. Based on this hypothesis, clinical trials for the development of drugs to treat valve stenosis are being conducted by administering anti-hyperlipidemic and osteoporosis medications such as PCSK9 inhibitors, niacin, alendronic acid, etc., but these attempts are presumed to be aimed at expanding the indications of existing drugs rather than specific valve stenosis treatment drugs, and clinical trials using some lipid-lowering drugs have reported no effect.

Korean Patent Publication No. 2018-0065224 relates to a pharmaceutical composition for treating or alleviating pulmonary fibrosis, and discloses a pharmaceutical composition for treating or alleviating pulmonary fibrosis characterized by comprising paquinimod, tasquinimod, laquinimod or a pharmaceutically acceptable salt thereof as an active ingredient. Korean Patent Publication No. 2018-0065223 relates to a pharmaceutical composition for treating or alleviating asthma, and discloses a pharmaceutical composition for treating or alleviating asthma characterized by comprising paquinimod or a pharmaceutically acceptable salt thereof as an active ingredient. Korean Patent No. 1855195 discloses a method for producing quinoline-3-carboxamide.

However, the effect of the present invention on preventing or treating the valvular calcification has not yet been disclosed.

### DETAILED DESCRIPTION OF THE INVENTION

### Summary

In order to solve the above problems, the purpose of the present invention is to confirm that the compound of Formula 1 or a salt thereof inhibits the expression of calcification and related protein indicators in valvular interstitial cells, and to provide a composition for inhibiting valvular calcification comprising the compound of Formula 1 or a salt thereof as an active ingredient.

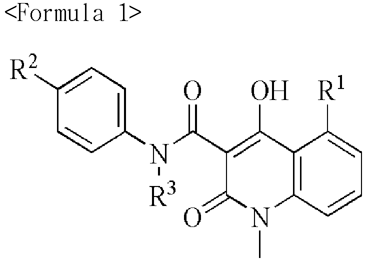

### Technical Solution

In order to solve the above problem, the present invention provides a pharmaceutical composition for preventing or treating the valvular calcification, comprising a compound of Formula 1 or a pharmaceutically acceptable salt thereof as an active ingredient.

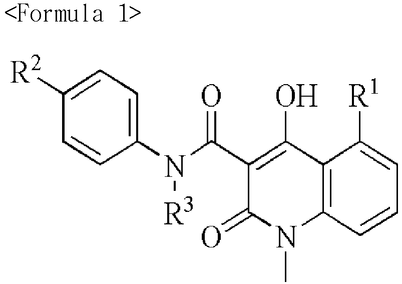

Wherein: R¹ is an ethyl group; a methoxy group; or chlorine (Cl), R² is hydrogen or carbon trifluoride (CF₃), and R³ is a methyl group or an ethyl group.

More preferably, the compound of Formula 1 may be at least one selected from the group consisting of paquinimod, tasquinimod, and laquinimod.

The valvular calcification is at least one selected from the group consisting of valvular disease, heart failure, myocardial ischemia, acute coronary syndrome, angina pectoris, and aortic valve stenosis, and the composition may inhibit OPN (Osteopontin) and RUNX2 (runt-related transcription factor 2), which are calcification indicators.

The compound of Formula 1 is administered in an amount of 0.001 to 10 g per day, and the composition may additionally comprise a component that inhibits valvular calcification.

As another example, the present invention provides a food composition for preventing or alleviating calcification, comprising a compound of Formula 1 or a food-wise acceptable salt thereof as an active ingredient.

### Effects of the Invention

The present invention relates to a composition for preventing or treating valvular calcification, and the compound of Formula 1 or a salt thereof can be used as an agent for preventing or treating various valvular calcification diseases.

### BRIEF DESCRIPTION OF THE DRAWINGS

Fig. 1 shows the calcification inhibitory effect of paquinimod according to the present invention in valvular interstitial cells. CM: control medium; PCM: pro-calcifying medium
Fig. 2 shows the calcification inhibitory effect of tasquinimod according to the present invention in valvular interstitial cells. CM: control medium; PCM: pro-calcifying medium
Fig. 3 shows the effect of reducing expression of valvular calcification markers of paquinimod according to the present invention in valvular interstitial cells.

### DETAILED DESCRIPTION

Hereinafter, preferred embodiments of the present invention will be described in detail. Additionally, many specific details, such as specific components, are shown in the following description, but these are provided only to help a more general understanding of the present invention, and it will be apparent to one skilled in the art that the present invention can be practiced without these specific details. And, in describing the present invention, if it is determined that a detailed description of a related known function or configuration may unnecessarily obscure the gist of the present invention, the detailed description is omitted.

In the present invention, the term "prevention" means inhibiting or delaying the occurrence of a disease from its cause.

In this specification, "treatment" means stopping the progression of damage by suppressing the progression and/or worsening of symptoms even if it doesn't heal completely, or leading toward healing by improving some or all of the symptoms.

In the present invention, the term "alleviation" refers to any act by which symptoms are alleviated or beneficially changed.

In order to achieve the purpose of the present invention, the present invention provides a pharmaceutical composition for preventing or treating valvular calcification, comprising a compound of Formula 1 or a pharmaceutically acceptable salt thereof as an active ingredient.

The compound of Formula 1 may be at least one selected from the group consisting of paquinimod, tasquinimod, and laquinimod.

Paquinimod is a compound with Formula 2 and is a Cas No. 248282-01-1 compound. Paquinimod was developed as a treatment for SLE (systemic lupus erythematosus), an autoimmune disease.

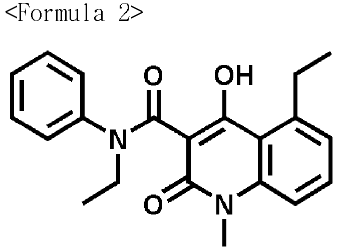

Tasquinimod is a compound having the structure of Formula 3 and is a Cas No. 254964-60-8 compound. Tasquinimod was developed as an anticancer agent and was primarily studied for prostate cancer.

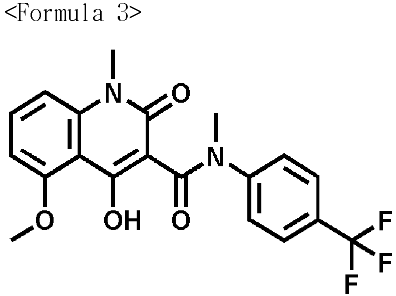

Laquinimod is a compound having Formula 4 and is a Cas No. 248281-84-7 compound. Laquinimod was developed as an immunomodulator and has been studied for multiple sclerosis.

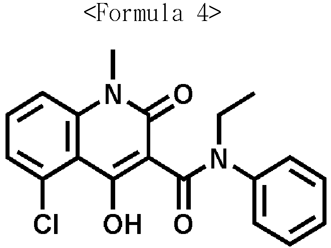

The compound of Formula 1 of the present invention may comprise a pharmaceutically acceptable salt form, which may be prepared by a method conventional in the art. For example, it forms acid salts with inorganic acids such as hydrochloric acid, hydrogen bromide, sulfuric acid, sodium bisulfate, phosphoric acid, and carbonic acid, or with organic acids such as formic acid, acetic acid, oxalic acid, benzoic acid, citric acid, tartaric acid, gluconic acid, gestic acid, fumaric acid, lactobionic acid, salicylic acid, or acetylsalicylic acid (aspirin), or reacting with alkali metal ions such as sodium and potassium to form metal salts thereof, or reacting with ammonium ions to form another form of pharmaceutically acceptable salt.

In one example of the present invention, valvular calcification is a type of calcification that occurs in the valve, and a general term for a calcification phenomenon that occurs particularly when excessive collagen is infiltrated into valvular interstitial cells and matrix proteins are destroyed, and includes various valve-related diseases due to loss of homeostasis of valvular interstitial cells. Preferably, but not limited to, at least one selected from the group consisting of valvular disease, heart failure, myocardial ischemia, acute coronary syndrome, angina pectoris, and aortic valve stenosis.

In another example of the present invention, the compound of Formula 1 is characterized by inhibiting OPN (Osteopontin) and RUNX2 (runt-related transcription factor 2), which are markers of calcification.

The composition of the present invention can be prepared into any form of a composition by further comprising suitable carriers, excipients and diluents commonly used in the preparation of compositions, and can preferably be manufactured in the form of a pharmaceutical composition or a (health functional) food composition, but is not limited thereto.

The pharmaceutical composition of the present invention can be formulated in the form of oral formulations such as powders, granules, tablets, capsules, suspensions, emulsions, syrups, aerosols, etc., and in the form of external preparations, suppositories, and sterile injection solutions according to conventional methods, more preferably in the form of a powder, tablet, capsule, injection, or liquid.

Such formulation can be carried out by methods commonly practiced in the pharmaceutical field, and can be preferably formulated according to each disease or ingredient using the method disclosed in Remington's Pharmaceutical Science, Mack Publishing Company, Easton PA.

Carriers, excipients and diluents that may be included in the pharmaceutical composition comprise lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methylhydroxybenzoate, propylhydroxybenzoate, talc, magnesium stearate, and mineral oil.

When formulating, it can be prepared by additionally using diluents or excipients such as fillers, bulking agents, binders, wetting agents, disintegrants, and surfactants that are commonly used.

Solid preparations for oral administration comprise tablets, pills, powders, granules, capsules, etc., and these solid preparations are prepared by mixing at least one excipient, such as starch, calcium carbonate, sucrose or lactose, gelatin, etc. Additionally, in addition to simple excipients, lubricants such as magnesium stearate and talc are also used.

Liquid preparations for oral administration include suspensions, solutions, emulsions, and syrups. In addition to commonly used simple diluents such as water and liquid paraffin, they may contain various excipients such as wetting agents, sweeteners, flavoring agents, and preservatives. Formulations for parenteral administration include sterile aqueous solutions, non-aqueous solutions, suspensions, emulsions, lyophilized preparations, and suppositories. Non-aqueous and suspending agents that can be used include propylene glycol, polyethylene glycol, vegetable oils such as olive oil, and injectable esters such as ethyl oleate. Suppository bases that can be used include witepsol, macrogol, tween, cocoa butter, laurin butter, and glycerogelatin.

The pharmaceutical composition of the present invention can be administered orally or parenterally (e.g., intravenously, subcutaneously, intraperitoneally, or topically) depending on the intended method, and the desired dosage varies depending on the patient's condition and weight, degree of disease, drug form, route of administration, and period of administration, but can be appropriately selected by those skilled in the art. However, for a desirable effect, the pharmaceutical composition of the present invention may be included in an amount of 0.001 to 99.9 wt%, preferably 0.1 to 99 wt%, per day. The daily dosage may be about 0.01 to 1,000 mg/kg, preferably 100 to 300 mg/kg. Depending on the need, the compound of Formula 1 may be administered in a daily dose of 0.001 to 10 g, but is not limited thereto. Also, in a composition according to one embodiment of the present invention, the composition may comprise an additional ingredient useful for preventing, alleviating or treating calcification, and the additional ingredient may comprise any ingredient known to be effective against calcification, comprising compounds and natural products.

As another example, the present invention provides a food composition for preventing or alleviating calcification, comprising a compound of Formula 1 or a food-wise acceptable salt thereof as an active ingredient.

The (health functional) food composition to which the composition can be added can be manufactured in the form of a powder, granule, tablet, capsule, or beverage. For example, there are various general foods, beverages, gum, tea, vitamin complexes, or health supplements.

The food composition of the present invention may comprise not only allithiamine, fursultiamine, benfotiamine or salts thereof, but also ingredients commonly added during food manufacturing, such as proteins, carbohydrates, fats, nutrients, seasonings and flavoring agents. Examples of the carbohydrates mentioned above are monosaccharides, e.g., glucose, fructose, etc.; disaccharides, such as maltose, sucrose, oligosaccharides, etc.; and common sugars such as polysaccharides, for example dextrins, cyclodextrins, etc., and sugar alcohols such as xylitol, sorbitol, and erythritol. Natural flavoring agents [thaumatin, stevia extracts (e.g., rebaudioside A, glycyrrhizin, etc.]) and synthetic flavoring agents (saccharin, aspartame, etc.) can be used as flavoring agents. For example, when the food composition of the present invention is manufactured as a drink, in addition to allithiamine, fursultiamine, benfotiamine or a salt thereof, citric acid, liquid fructose, sugar, glucose, acetic acid, malic acid, juice, eucommia extract, jujube extract, licorice extract, etc. may be additionally included.

Additionally, the composition can be added to food or beverages for the purpose of preventing diseases. At this time, the amount of the effective ingredient in the food or beverage can be added at 0.001 to 15 wt% of the total food weight. The food composition may be added in a proportion of 0.002 to 5 g, preferably 0.03 to 1 g, per 100 g, but is not limited thereto.

The advantages and features of the present invention and the methods for achieving them will become apparent with reference to the embodiments described in detail below. However, the present invention is not limited to the embodiments disclosed below, but may be implemented in various different forms. These examples are provided solely to make the disclosure of the present invention complete and to fully inform those skilled in the art of the scope of the invention, and the present invention is defined only by the scope of the claims.

### <Example 1> Experimental method

### 1. Culture and induction of calcification of porcine valvular interstitial cells

To isolate porcine valvular interstitial cells, porcine heart valves were treated with collagenase for 15 minutes, after which endothelial cells were removed and cultured again for 24 hours. The cells obtained through centrifugation are valvular interstitial cells and were cultured in DMEM (high-glucose; Gibco) medium containing 0.1% amphotericin B, 1% penicillin-streptomycin, and 5% FBS.

To induce calcification, cells were seeded at a density of 1×10⁴/ml in a 96-well culture vessel, and calcification was induced for a total of 10 days. Control medium (CM) used DMEM containing 0.1% amphotericin B, 1% penicillin-streptomycin, and 5% FBS and the pro-calcifying medium (PCM) was a control medium with 2 mM NaH₂PO₄ added. The medium was replaced every 2 days.

To observe if anti-calcification substances inhibit calcification, 10 µM or 100 µM paquinimod or 10 µM tasquinimod were treated, and 10% DMSO (Sigma-Aldrich) was treated as a control, and then cultured in a cell incubator at 37°C with a 5% CO₂ concentration.

### 2. Calcification staining

To observe calcification deposits in the valvular interstitial cells, calcification was observed using a 40 mM Alizarin Red S Staining Kit (Sciencell, Carlsbad, CA). After removing the medium, the cells were washed with PBS buffer, fixed with 4% paraformaldehyde for 20 minutes, and then washed with dH₂O. Afterwards, it was stained with Alizarin Red Solution for 20 minutes at room temperature (in the dark), washed 2 to 4 times with 3rd distilled water, and observed and photographed under a microscope.

To quantify Alizarin Red stain, after washing twice with PBS, it was fixed with cold 70% ethanol at 4°C for 1 hour, and after washing with dHzO, the sample was treated with 10% CPC (cetylpyridinium chloride, Sigma-Aldrich) for 1 hour (shaken and in the dark), transferred to a new measuring container, and measured for absorbance at 550 nm.

### 3. Western blot analysis

After seeding cells at 5×10⁴/mL in a 6-well plate, calcification was induced for a total of 10 days. Western blot analysis was performed to determine whether there were changes in calcification markers (Osteopontin, RUNX2). To lyse the cells, 1X RIPA buffer was added to each well and lysed for 10 minutes. Cells were scraped with a scraper and centrifuged at 14,000 rpm, 4°C for 15 minutes. Protein quantification of the obtained cell lysates was measured using Bio-Rad DC protein reagent. The analysis was performed by equally loading 20 ug of protein. After reacting with primary antibodies against osteopontin (1:1,000), RUNX2 (1:1,000), or beta-actin (1:3,000) at 4°C for 24 hours, the cells were washed 3 to 5 times for 10 minutes each with 1X TBS-T buffer. Secondary antibodies were reacted at a ratio of 1:10,000 at room temperature for 1 hour, and then washed 3 to 5 times for 10 minutes each with 1X TBS-T buffer. Protein bands were identified using Amersham imager 680 after ECL solution treatment.

### <Example 2> Effect of anticalcification substance on valvular calcification

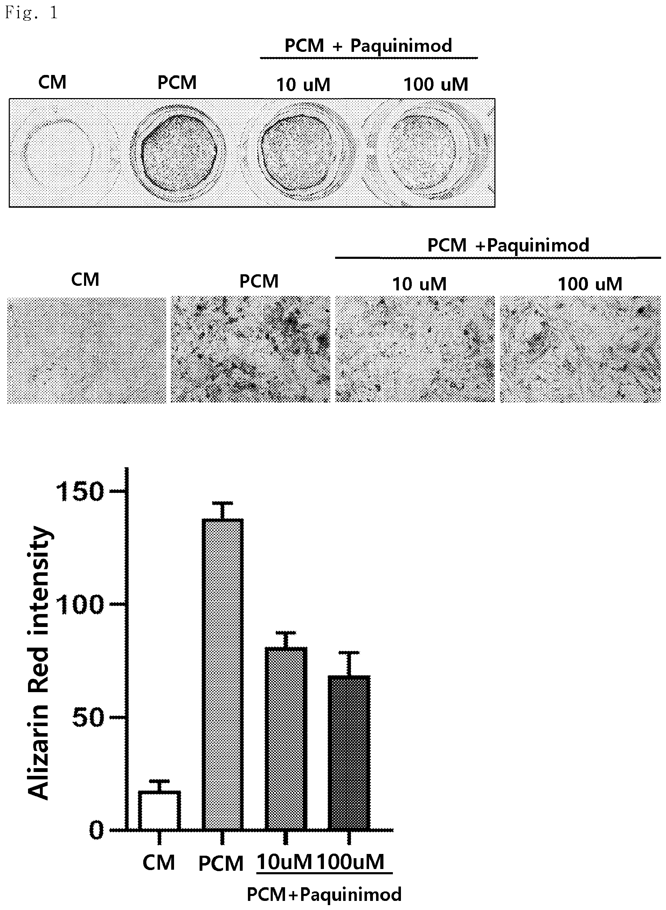

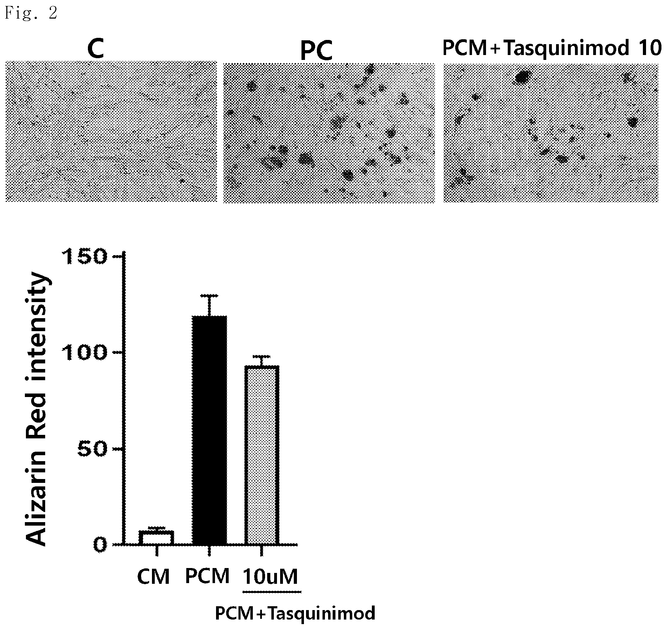

Interstitial cells were isolated from porcine heart valves and the inhibitory effect of paquinimod or tasquinimod on valvular calcification was confirmed according to the concentration. Porcine valve-derived cells were induced to calcify by NaH₂PO₄, and the degree of valvular calcification was measured after treatment with paquinimod or tasquinimod at different concentrations under the same conditions. As a result, inhibition of valvular calcification in a concentration-dependent manner in valvular interstitial cells treated with 10 µM or more of paquinimod was confirmed through Alizarin red staining and CPC analysis (Fig. 1). Also, it was confirmed that tasquinimod had an inhibitory effect on valvular calcification at a concentration of 10 µM (Fig. 2). These results showed that paquinimod or tasquinimod can be applied as new drugs to suppress valvular calcification and resulting valvular stenosis, which are intractable heart diseases.

### <Example 3> Confirmation of reduction in valvular calcification by calcification inhibitor

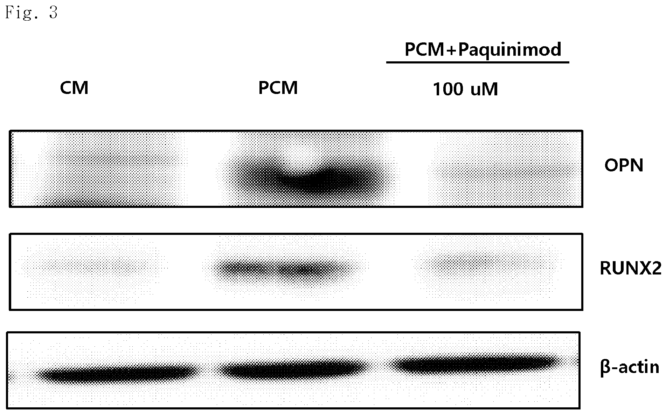

Osteopontin (OPN) or RUNX2 (Runt-related transcription factor2), which are biomarkers of calcification, increased in the calcification-induced experimental group compared to the control group, and the expression of osteopontin or RUNX2 was significantly reduced in the paquinimod-treated group compared to the calcification-induced experimental group (Fig. 3).

## Claims

1. A pharmaceutical composition for preventing or treating the valvular calcification, comprising a compound of Formula 1 or a pharmaceutically acceptable salt thereof as an active ingredient: 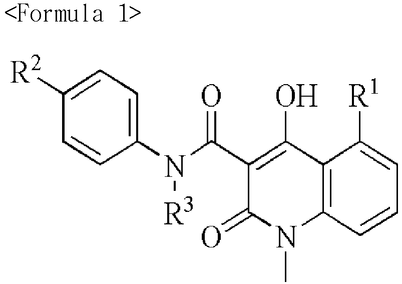 Wherein: R¹ is an ethyl group; a methoxy group; or chlorine (Cl), R² is hydrogen or carbon trifluoride (CF3), and R³ is a methyl group or an ethyl group.

2. The pharmaceutical composition for preventing or treating the valvular calcification according to claim 1, wherein the compound of Formula 1 is at least one selected from the group consisting of paquinimod, tasquinimod, and laquinimod.

3. The pharmaceutical composition for preventing or treating the valvular calcification according to claim 1, wherein the valvular calcification is at least one selected from the group consisting of valvular disease, heart failure, myocardial ischemia, acute coronary syndrome, angina pectoris, and aortic valve stenosis.

4. The pharmaceutical composition for preventing or treating the valvular calcification according to claim 1, wherein the compound of Formula 1 is administered in an amount of 0.001 to 10 g per day.

5. The pharmaceutical composition for preventing or treating the valvular calcification according to claim 1, wherein the composition may additionally comprise a component inhibiting valvular calcification.

6. A food composition for preventing or alleviating calcification, comprising a compound of Formula 1 or a food-wise acceptable salt thereof as an active ingredient: 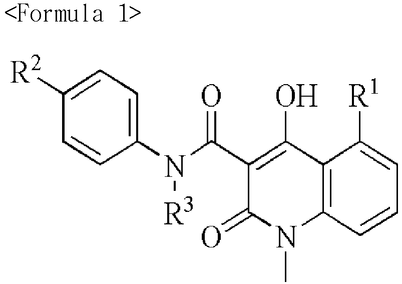 Wherein: R¹ is an ethyl group; a methoxy group; or chlorine (Cl), R² is hydrogen or carbon trifluoride (CF3), and R³ is a methyl group or an ethyl group.

7. The food composition for preventing or treating the valvular calcification according to claim 6, wherein the compound of Formula 1 is at least one selected from the group consisting of paquinimod, tasquinimod, and laquinimod.

8. The food composition for preventing or treating the valvular calcification according to claim 6, wherein the valvular calcification is at least one selected from the group consisting of valvular disease, heart failure, myocardial ischemia, acute coronary syndrome, angina pectoris, and aortic valve stenosis.

9. The food composition for preventing or treating the valvular calcification according to claim 6, wherein the compound of Formula 1 is administered in an amount of 0.001 to 10 g per day.

10. The food composition for preventing or treating the valvular calcification according to claim 6, wherein the composition may additionally comprise a component inhibiting valvular calcification.

11. A method for preventing or treating the valvular calcification comprising a step of administering a compound of Formula 1 or a pharmaceutically acceptable salt thereof: 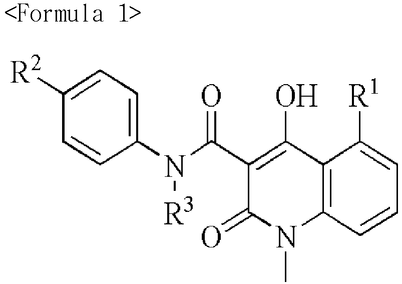 Wherein: R¹ is an ethyl group; a methoxy group; or chlorine (Cl), R² is hydrogen or carbon trifluoride (CF3), and R³ is a methyl group or an ethyl group.
